# EUROPÄISCHE PATENTSCHRIFT

(11) **EP 2 607 345 B1**
(45) Veröffentlichungstag und Bekanntmachung des Hinweises auf die Patenterteilung: **12.08.2015**
(21) Anmeldenummer: 12007616.1
(22) Anmeldetag: 09.11.2012
(51) Int. Cl.: C07C 45/62, C07C 45/00

(54) **Verfahren zur Herstellung von cyclischen Ketonen**
Method for producing cyclic ketones
Procédé de fabrication de cétones cycliques

(30) Priorität: 24.12.2011 EP 11010207
(43) Veröffentlichungstag der Anmeldung: 26.06.2013
(73) Patentinhaber: Allessa GmbH, 60386 Frankfurt am Main (DE)
(72) Erfinder: Neumann, Doris, 63069 Offenbach (DE); Ritzer, Joachim, 63517 Rodenbach (DE); Effenberger, Gunther, 61118 Bad Vilbel (DE)
(74) Vertreter: Ackermann, Joachim

(56) Entgegenhaltungen:
- EP-A1- 0 913 376
- JP-A- 11 060 534
- US-A- 4 409 401

## Beschreibung

Die vorliegende Erfindung betrifft ein Verfahren zur Herstellung von cyclischen Ketonen, insbesondere von Cyclohexanonen, durch katalytische Hydrierung von entsprechenden Phenolen.

Es ist bekannt, dass sich Phenole katalytisch zu cyclischen Ketonen hydrieren lassen. So beschreibt US-A-2,829,166 die Hydrierung von Phenolen in Gegenwart von Palladium-Katalysatoren zu den entsprechenden Cyclohexanonen. Aus DE 29 09 780 A1 ist ein Verfahren zur Hydrierung von p-tert.-Amylphenol bekannt. In der EP 731 075 A1 wird ein Verfahren zur Herstellung von substitutierten Cyclohexanonen offenbart. Desweiteren offenbaren EP 889 019 A1 und EP 890 565 A1 Verfahren zur Herstellung von Cyclohexanonen durch Hydrierung der entsprechenden Phenole in Gegenwart von Alkanen oder von Wasser als Lösungsmittel. Der Einsatz von aliphatischen Alkoholen als Lösungsmittel wird in diesen Schriften nicht offenbart.

Gute Selektivitäten an Cyclohexanonen erhält man im Allgemeinen bei Temperaturen von 120 - 250°C und einem Wasserstoffdruck von 1 - 20 bar. Dabei kann es vorteilhaft sein, die Pd/C-Katalysatoren mit einem Alkali- oder Erdalkalimetallcarbonat oder sonstigen basischen oder neutralen Salzen zu behandeln oder dem Hydriergemisch basische bzw. neutrale Salze zuzusetzen.

Bei den zur Phenolhydrierung einsetzbaren Lösungsmitteln ist man allerdings eingeschränkt. Beschrieben ist die Hydrierung in Substanz (vergl. US-A-2,829,166), was aber nur bei Phenolen, die im Bereich der Hydriertemperaturen schmelzen, möglich ist. An weiteren Lösungsmitteln sind lediglich Ether (vergl. EP 731 075 A1), spezielle Alkane wie z.B. Methylcyclohexan (vergl. EP 889 019 A1) oder Ether (vergl. EP 890 565 A1) bekannt. Ferner wird von M. Wydra und H. Vogel in Chemie Ing. Techn. 74, 800-804, 2002, die katalytische Hydrierung von Phenolen in Aromaten als Lösungsmittel, wie z.B. in Toluol und Xylol, beschrieben.

Aus JP 11-060534 A ist ein Verfahren zur Herstellung von Hydroxycyclohexanonen bekannt, bei dem substituierte Polyphenole, wie Resorcin, Hydrochinon oder Pyrogallol, in Gegenwart eines Alkalimetall enthaltenden Palladiumkatalysators in einem gesättigten einwertigen C₃-C₁₂-Alkohol hydriert werden. Der Pd-Katalysator kann beispielsweise durch Behandlung mit Alkalimetallhydroxiden erzeugt werden.

EP 0 913 376 A1 offenbart ein Verfahren zur Herstellung von Cyclohexanonen aus den entsprechenden Phenolen durch partielle Hydrierung. Das durch Hydrierung erhaltene Reaktionsgemisch vor der Isolierung des Cyclohexanons mit Sulfiermitteln behandelt. Für die Durchführung der Hydrierung werden aromatische Lösungsmittel, gesättigte Kohlenwasserstoffe, Ester, Ether und Alkohole vorgeschlagen.

Es ist bekannt, dass beim Einsatz von polareren Lösungsmitteln wie z.B. Alkoholen, die den Anwendungsbereich dieser Reaktion deutlich erweitern würden, wesentlich geringere Umsätze und Selektivitäten auftreten. So beschrieben z.B. Tagaki et al. in Energy & Fuels 13 (6), 1191-1196, 1999, die Kernhydrierung verschiedener Aromaten und fanden, dass im Falle der Katalyse durch Pt/C Alkohole die Reaktivität stark herabsetzen. Higashijima und Nishimura beschrieben in Bull.Chem. Soc. Jpn. 65, 2955-2959, 1992 Untersuchungen zur Kernhydrierung von Phenol und Kresolen an Pd/C-Katalysatoren in verschiedenen Lösungsmitteln und fanden, dass in Alkoholen sowohl die Hydrierrate als auch die Selektivität zu Gunsten der Cyclohexanone, im Vergleich zur Hydrierung in Alkanen oder in Substanz, deutlich herabgesetzt waren. Dieser Effekt wurde beim Einsatz kommerziell erhältlicher Pd/C-Katalysatoren für unterschiedliche primäre, sekundäre und tertitäre Alkohole mit größer gleich 3 Kohlenstoffatomen gefunden; bei sauer behandeltem Pd/C-Katalysator war der Effekt etwas abgeschwächt.

In der US-A-4,409,401 wird ein Hydrierverfahren von Phenolen offenbart, bei dem ein gezielt mit Schwefel vergifteter Palladium-Katalysator eingesetzt wird. Die Herstellung des Katalysators erfolgt durch oxidative und anschließend durch reduktive Behandlung. In diesem Dokument wird ausgeführt, dass während der Hydrierung vorteilhaft basische Substanzen zugegen sind, beispielsweise Na-Carbonat. Die Umsetzung kann in Gegenwart eines Verdünnungsmittels erfolgen. Als Beispiele dafür werden gesättige und aromatische Kohlenwasserstoffe, Alkohole oder das angestrebte Verfahrensprodukt genannt.

EP-A-822,173 offenbart ein Verfahren zur Herstellung von 1,3-Cyclohexandionen. Ausgehend von 1,3-Bisphenolen wird ein Transferhydrierverfahren durchgeführt, bei dem ein Wasserstoffdonor, typischerweise ein sekundärer Alkohol oder Ameisensäure bzw. eines ihrer Salze, das Ausgangsprodukt in Gegenwart eines PalladiumKatalysators hydriert. Eine klassische Hydrierung mit Hilfe von Wasserstoff wird nicht offenbart.

US-A-2010/041714 betrifft die Herstellung von Bisarylamino-tetralinen. In einem Ausführungsbeispiel wird die Herstellung eines Vorproduktes 5-Hydroxy-3,4-dihydro-2H-naphthalin-1-on beschrieben. Dieses erfolgt durch Hydrierung von 1,5-Dihydroxynaphthalin in Isopropanol und wässeriger Natronlauge in Gegenwart eines auf Kohle geträgerten Palladium-Katalysators.Die Anteile an Palladium und an Natronlauge, bezogen auf das eingesetzte Bisphenol, liegen bei diesem Beispiel recht hoch.

Überraschend wurde nun gefunden, dass sich Phenole mit einer Hydroxylgruppe in längerkettigen aliphatischen und/oder cycloaliphatischen Alkoholen mit hoher Selektivität bei hohem Umsatz zu den entsprechenden cyclischen Ketonen hydrieren lassen, wenn dabei ein ausgewählter Katalysator eingesetzt wird.

Die vorliegende Erfindung betrifft ein Verfahren zur Hydrierung von Phenolen mit einer Hydroxylgruppe zu cyclischen Ketonen durch Umsetzung der Phenole mit Wasserstoff in Gegenwart eines auf Kohlenstoff geträgerten Palladium enthaltenden Katalysators, in einer alkoholischen Lösung, wobei ein mindestens drei Kohlenstoffatome enthaltender aliphatischer und/oder ein cycloaliphatischer Alkohol nach Anspruch 1 eingesetzt wird und der auf Kohlenstoff geträgerte Palladium enthaltende Katalysator vor dem Einsatz mit einem neutralen oder basischen Salz, vorzugsweise einem Alkali- und/oder Erdalkali- und/oder Ammoniumsalz behandelt worden ist und/oder die Hydrierung in Gegenwart eines geträgerten Palladium enthaltenden Katalysators und eines neutralen oder basischen Salzes erfolgt, mit der Massgabe, dass der Palladium enthaltende Katalysator keine anorganischen und/oder organischen Schwefelverbindungen enthält.

Bei der Durchführung des erfindungsgemäßen Verfahrens hat sich herausgestellt, dass man gute Selektivitäten an cyclischen Ketonen bei hohem Umsatz der Phenole im Allgemeinen bei Temperaturen von 80 bis 250°C, vorzugsweise von 120 bis 250°C, besonders bevorzugt bei 90 bis 200 °C und ganz besonders bevorzugt bei 100 bis 180°C erhält.

Das erfindungsgemäße Verfahren kann bei unterschiedlichen Wasserstoffdrucken durchgeführt werden. Die Selektivität und der Umsatz der Reaktion sind am höchsten bei einem Wasserstoffdruck von 0,5 - 20 bar. Bevorzugt hydriert man bei 0,5 - 15 bar, besonders bevorzugt bei 1 - 10 bar.

Das erfindungsgemäße Verfahren ist **dadurch gekennzeichnet,** dass man ein einwertiges Phenol in einem ausgewählten aliphatischen und/oder cycloaliphatischen Alkohol nach Anspruch 1 mit mindestens drei Kohlenstoffatomen löst, mit einem auf Kohlenstoff geträgerten Pd-enthaltenden Katalysator und einem neutralen oder basischen Salz versetzt oder mit einem auf Kohlenstoff geträgerten Pd-enthaltenden Katalysator versetzt, der mit einem neutralen oder basischen Salz modifiziert worden ist, und das einwertige Phenol bei Reaktionstemperatur gegebenenfalls unter Druck mit Wasserstoff umsetzt. Die Umsetzung erfolgt vorzugsweise in einem Autoklaven.

Als alkoholische Lösungsmittel werden geradkettige oder verzweigte sekundäre oder tertiäre aliphatische Alkohole mit mindestens drei Kohlenstoffatomen eingesetzt. Dabei handelt es sich um geradkettige oder verzweigte sekundäre oder tertiäre aliphatische Alkohole mit mindestens drei Kohlenstoffatomen Diese Alkohole können gerad- oder verzweigtkettig oder auch cyclisch sein. Ganz besonders bevorzugt werden sekundäre oder tertiäre Alkohole mit drei bis acht Kohlenstoffatomen verwendet. Beispiele hierfür sind Isopropanol, 2-Butanol, tert-Butanol, 2-Pentanol, 3-Pentanol, 2-Methylbutan-2-ol, 3-Methylbutan-2-ol, Cyclopentanol, Cyclohexanol, 2-Methylpentan-2-ol, 2-Methylpentan-3-ol, 3-Methylpentan-2-ol, 3-Methylpentan-3-ol, 4-Methylpentan-2-ol, 2,3-Dimethyl-2-butanol, 3,3-Dimethyl2-butanol, 2-Hexanol, 3-Hexanol, 2,2-Dimethyl-3-pentanol, 2,3-Dimethyl-3-pentanol, 2,4-Dimethyl-3-pentanol, 4,4-Dimethyl-2-pentanol, 3-Ethyl-3-pentanol, 2-Methyl-2-hexanol, 2-Methyl-3-hexanol, 5-Methyl-2-hexanol, 2-Heptanol, 3-Heptanol, isomere Methylcyclohexanole, 2-Octanol, 3-Octanol, 4-Methylheptan-3-ol, 6-Methylheptan-2-ol, 2-Ethylhexanol und 3,7-Dimethyl-3-octanol.

Beispiele für cycloaliphatische Alkohole sind Cyclohexanol oder Methylcyclohexanol.

Die Verdünnung des Phenols im entsprechenden alkoholischen Lösungsmittel wählt man in der Regel zu 10 bis 95 %ig, bevorzugt zu 20 bis 90%ig, besonders bevorzugt zu 25 bis 85%ig.

Neben dem alkoholischen Lösungsmittel kann das erfindungsgemäß eingesetzte Reaktionsgemisch noch weitere mit den geradkettigen oder verzweigten sekundären oder tertiären aliphatischen Alkoholen mit mindestens drei Kohlenstoffatomen mischbare Flüssigkeiten aufweisen. So kann beispielsweise ein geringer Anteil an Wasser zugegen sein, beispielsweise bis zu 20 Gew. % an Wasser, vorzugsweise zwischen 0,1 und 10 Gew. % an Wasser, bezogen auf die Gesamtmenge des Lösungsmittels. Die Menge an weiteren mit den Alkoholen mischbaren Flüssigkeiten, insbesondere Wasser, ist im Einzelfall so auszuwählen, dass sich ein einphasiges System ergibt und keine Entmischung erfolgt. Die Anwesenheit von Wasser ist bevorzugt, insbesondere wenn die Hydrierung in Gegenwart eines neutralen oder basischen Salzes vorgenommen wird.

Im erfindungsgemäßen Verfahren kommen auf Kohlenstoff geträgerte Palladium enthaltende Katalysatoren zum Einsatz. Als Träger eignen sich Kohlenstoff bzw. Graphit. Trägermaterialien für diese Katalysatoren sind dem Fachmann bekannt und werden in der Regel in feinverteilter Form verwendet, die gegebenenfalls zu Pellets verpresst sein können. Besonders bevorzugt wird Aktivkohle als Trägermaterial eingesetzt.

Das katalytisch aktive Metall ist Palladium oder eine Kombination von Palladium mit anderen Metallen, beispielsweise mit Platin oder Rhodium.

Der Katalysator kann neben dem katalytisch aktiven Metall noch mit weiteren Komponenten dotiert sein, beispielsweise mit Alkali- oder Erdalkalimetallen. Dabei handelt es sich im Allgemeinen um katalytisch aktive Co-Komponenten.

Es werden Palladium enthaltenden Katalysatoren weisen keine anorganischen und/oder organischen Schwefelverbindungen aufweisen, wie anorganische Sulfide oder Hydrosulfide, oder wie organische Sulfide, Disulfide, Thiosäuren oder Merkaptane sowie heterocyclische Schwefelverbindungen.

Für den auf Kohlenstoff geträgerten Pälladium-Katalysator (nachstehend "Pd/C-Katalysator") können beliebige Kohleträger eingesetzt werden. Diese Pd/C-Katalysatoren sind handelsüblich.

Der auf Kohlenstoff geträgerte Katalysator enthält in der Regel von 1 bis. 15 Gew. % an katalytisch aktivem Metall, bevorzugt von 1 bis 10 Gew. % und ganz besonders bevorzugt von 1 bis 5 Gew. %. Bevorzugt enthält er 1 - 10% Edelmetall, besonders bevorzugt 1 - 5% Edelmetall. Die auf Kohlenstoff geträgerten Katalysatoren können als Feststoffe, z.B. als Pulver oder als Pellets eingesetzt werden oder auch als wasserfeuchte Paste. Der oder die Katalysatoren liegen im Reaktionsgemisch suspendiert vor; die übrigen Bestandteile des Reaktionsgemisches, wie das oder die Lösungsmittel, das Phenol und das hydrierte Phenol liegen in der Regel als Einphasengemisch vor und bilden eine Lösung, die mit dem in der Gasphase vorliegenden Wasserstoff reagiert.

Bezogen auf das Phenol werden diese Katalysatoren in Mengen von 1 x 10⁻⁴ bis 1 Gew.-% (gerechnet jeweils als Pd-Metall) eingesetzt. Bevorzugt werden sie in Mengen von 1 x 10⁻³ bis 1 Gew.-% eingesetzt, besonders bevorzugt in Mengen von 1 x 10⁻³ bis 0,1 Gew.-%.

Der im erfindungsgemäßen Verfahren eingesetzte Katalysator wird vor dem Einsatz mit einem neutralen oder basischen Salz, in der Regel mit einer Lösung eines neutralen oder basischen Alkali-, Erdalkali- oder Ammoniumsalzes behandelt oder das neutrale oder basische Salz wird dem Hydriergemisch zugesetzt. In letzterem Fall ist es möglich auch vorher unbehandelte auf Kohlenstoff geträgerte Katalysatoren einzusetzen. Das neutrale oder basische Salz kann als Substanz dem auf Kohlenstoff geträgerten Katalysator zugesetzt werden oder vorzugsweise als Lösung in einem geeigneten Lösungsmittel, insbesondere als wässrige Lösung. Als neutrale oder basische Salze setzt man Metallsalze ein, bevorzugt Hydroxide, Carbonate, Hydrogencarbonate, Phosphate, Monohydrogenphosphate, Formiate, Acetate, Propionate oder Borate von Alkalimetallen, wie von Lithium, Natrium, Kalium, Rubidium oder Cäsium; oder von Erdalkalimetallen, wie von Beryllium, Magnesium, Calcium, Strontium oder Barium; oder von Ammonium. Besonders bevorzugt werden Hydroxide, Carbonate, Hydrogencarbonate, Phosphate, Monohydrogenphosphate, Acetate oder Borate von Lithium, Natrium, Kalium, Magnesium, Calcium, Strontium oder Ammonium eingesetzt. Beispiele sind Lithium-, Natrium-, Kalium- oder Ammoniumhydroxid, Natrium-, Kalium- oder Ammoniumhydrogencarbonat, Lithium-, Natrium-, Kalium-, Ammonium-, Magnesium-oder Calciumcarbonat, Lithium-, Natrium-, Kalium-, Ammonium- oder Magnesiumphosphat, Lithium-, Natrium-, Kalium-, Ammonium- oder Calciumformiat, Lithium-, Natrium-, Kalium-, Ammonium- oder Calciumacetat, Lithium-, Natrium-, Kalium-, Ammonium- oder Calciumpropionat oder Lithium-, Natrium-, Kalium- oder Ammoniumborat. Es können auch Gemische von neutralen oder basischen Salzen eingesetzt werden.

Unter einem neutralen oder basischen Salz ist im Rahmen dieser Erfindung ein Salz zu verstehen, das als 0,1 - 15%ige wässrige Lösung einen pH-Wert von 7 oder von größer als 7 aufweist.

Dieses dem Katalysator und/oder der Reaktionslösung zugesetzte neutrale oder basische Salz gibt man in Mengen von 0,001 bis 5 Mol-% bezogen auf eingesetztes Phenol zu. Bevorzugt gibt man das Salz in Mengen von 0,005 bis 5 Mol-% zu, besonders bevorzugt in Mengen von 0,005 bis 1 Mol-%. Die Behandlung oder Zugabe kann mit dem Salz in Lösung erfolgen, beispielsweise in Wasser oder in alkoholischen Lösungsmitteln.

Vorzugsweise setzt man das neutrale oder basische Salz als wässrige Lösung dem auf Kohlenstoff geträgerten Katalysator oder der Reaktionsmischung zu. Das Salz wird zur Herstellung der wässrigen Lösung im Allgemeinen in der 1 bis 1000-fachen Menge an Wasser, bezogen auf das Salz, aufgelöst. Der Anteil an Wasser sollte so gewählt werden, dass das Wasser mit dem Alkohol mit mindestens drei Kohlenstoffatomen eine homogene Mischung und kein Mehrphasensystem bildet.

Der im erfindungsgemäßen Verfahren eingesetzte auf Kohlenstoff geträgerte Palladium-Katalysator kann frisch sein oder es kann ein bereits ein- oder mehrmals verwendeter Katalysator eingesetzt werden. Es hat sich überraschenderweise gezeigt, dass sogar die Rückführung der eingesetzten Katalysatoren möglich ist, was bei nicht auf diese Weise behandelten Katalysatoren bzw. bei Reaktionsführung in anderen Lösungsmitteln nur mit erheblichem Verlust an Selektivität möglich war.

Als Phenole können im erfindungsgemäßen Verfahren beliebige Verbindungen eingesetzt werden, welche mindestens einen aromatischen Kern und damit kovalent verbunden eine Hydroxylgruppe aufweisen.

Bei den Phenolen kann es sich um ein- oder mehrkernige carbocyclische aromatische Verbindungen handeln, vorzugsweise um drei-, zwei- oder insbesondere einkerninge carbocyclische aromatische Verbindungen; oder es kann sich um ein- oder mehrkernige heterocyclische aromatische Verbindungen handeln, die vorzugsweise ein oder zwei Ringheteroatome ausgewählt aus der Gruppe Stickstoff, Sauerstoff und Schwefel oder Kombinationen davon aufweisen.

Diese carbocyclischen oder heterocyclischen aromatischen Verbindungen weisen eine phenolische Hydroxylgruppe auf, und gegebenenfalls einen oder mehrere Substituenten, beispielsweise Substituten ausgewählt aus der Gruppe der Alkyl-, Alkoxy-, Hydroxyalkyl-, Cycloalkyl-, Cycloalkoxy-, Aryl-, Aryloxy-, Aralkyl-, Aralkyloxy-, Carbonsäure-, Sulfonsäure-, Amino-, Nitro-, Carbonsäureester-, Carbonsäureamid-, Sulfonsäureester-, Sulfonsäureamid- und/oder Nitrilgruppen oder von Kombinationen von zwei oder mehreren dieser Gruppen bzw. Atome.

Es werden also carbocyclische oder heterocyclische aromatische Verbindungen mit nur einer phenolischen Hydroxylgruppe eingesetzt.

Beispiele für Alkylgruppen sind geradkettige oder verzweigte Alkylgruppen mit ein bis zehn Kohlenstoffatomen, wie Methyl, Ethyl, Propyl, Isopropyl, n-Butyl, sek.-Butyl, tert-Butyl, Pentyl, n-Hexyl, n-Heptyl, 2-Ethylhexyl, n-Octyl, n-Nonyl oder n-Decyl.

Beispiele für Alkoxygruppen sind solche mit geradkettigen oder verzweigten Alkylgruppen mit ein bis zehn Kohlenstoffatomen, wie Methoxy, Ethoxy, Isopropoxy, n-Butoxy, sek.-Butoxy, tert-Butoxy, Pentyloxy, n-Hexyloxy, n-Heptyloxy, 2-Ethylhexyloxy, n-Octyloxy, n-Nonyloxy oder n-Decyloxy.

Beispiele für Cycloalkylgruppen sind solche mit fünf oder sechs Ringkohlenstoffatomen, die ihrerseits wiederum substitutiert sein können, beispielsweise mit Alkylgruppen. Ein Beispiel für eine Cycloalkylgruppe ist Cyclohexyl.

Beispiele für Cycloalkoxygruppen sind solche mit fünf oder sechs Ringkohlenstoffatomen im Cycloalkylring, der seinerseits wiederum substitutiert sein kann, beispielsweise mit Alkylgruppen. Ein Beispiel für eine Cycloalkoxygruppe ist Cyclohexyloxy.

Beispiele für Arylgruppen sind solche mit sechs oder zehn Ringkohlenstoffatomen im Arylring, die ihrerseits wiederum substitutiert sein können, beispielsweise mit Alkylgruppen. Ein Beispiel für eine Arylgruppe ist Phenyl.

Ein Beispiel für eine Aralkylgruppe ist Benzyl, das seinerseits wiederum substitutiert sein kann, beispielsweise mit Alkylgruppen.

Ein Beispiel für eine Carbonsäureamidgruppe ist C₁-C₄-Acylamino, vorzugsweise Acetylamino.

Die carbocyclischen oder heterocyclischen aromatischen Verbindungen können neben aromatischen Ringen noch nicht-aromatische gesättigte oder ethylenisch ungesättigte Ringe aufweisen, welche mit den aromatischen Ringen annelliert sind oder mit den aromatischen Ringen über kovalente Bindungen verknüpft sind oder mit den aromatischen Ringen ein bi- oder polycyclisches System bilden.

Beispiele für besonders bevorzugt eingesetzte Phenole sind solche, die sich von Phenylgruppen, substitutierten Phenylgruppen, Naphthylgruppen, substituierten Naphthylgruppen, Anthracenylgruppen und substitutierten Anthracenylgruppen ableiten und die eine phenolische Hydroxylgruppen aufweisen.

Besonders bevorzugt werden Phenole der Formel (I) eingesetzt 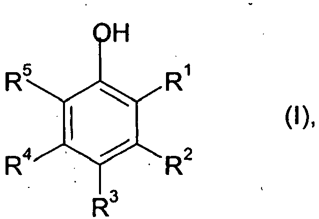 worin R¹, R², R³, R⁴, R⁵ unabhängig voneinander Wasserstoff, C₁-C₁₀-Alkyl, C₃-C₆-Cycloalkyl, C₆-C₁₀-Aryl, C₃-C₁₀-Heteroaryl mit O, N und/oder S als Ring-Heteroatome, C₆-C₁₀-Aryl-CH₂, C₆-C₁₀-Aryl-O, C₁-C₁₀-Alkoxy, C₃-C₈-Cycloalkoxy, N-C₁-C₄-Alkylamino, N,N-Di-C₁-C₄-alkylamino, C₁-C₄-Acylamino, COOR⁶ mit R⁶ = Wasserstoff, C₁-C₄-Alkyl oder -CH₂-R⁷ mit R⁷ = Hydroxyl, C₁-C₄-Alkyoxy, N-C₁-C₄-Alkylamino oder N, N-Di-C₁-C₄-alkylamino bedeuten.

Diese Phenole werden durch das erfindungsgemäße Verfahren in hoher Selektivität zu den entsprechenden Cyclohexanonen der allgemeinen Formel (II) hydriert 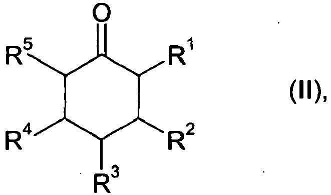 wobei R¹, R², R³, R⁴, R⁵ die oben definierte Bedeutung haben.

In den Formeln (I) und (II) stehen R¹, R², R³, R⁴, R⁵ unabhängig voneinander bevorzugt für Wasserstoff, C₁-C₆-Alkyl, C₁-C₆-Alkoxy oder C₁-C₄-Acylamino, wobei bevorzugt 1 bis 4 der Reste R¹ bis R⁵ von Wasserstoff verschieden sind.

Besonders bevorzugt stehen R¹, R², R⁴ und R⁵ für Wasserstoff und R³ für C₁-C₁₀-Alkyl, C₁-C₁₀-Alkoxy oder C₁-C₄-Acylamino.

Das beschriebene Verfahren kann in beliebigen Reaktoren durchgeführt werden. Bevorzugte Beispiele dafür sind Rühr- oder Schlaufenreaktoren.

Die Hydrierzeiten des beschriebenen Verfahrens liegen im Allgemeinen zwischen ein und drei Stunden. Im Einzelfall können aber auch kürzere oder längere Reaktionszeiten verwendet werden.

Die erreichten Selektivitäten an cyclischen Ketonen nach dem erfindungsgemäßen Verfahren liegen häufig oberhalb von 94%, teils bei bis zu 96%.

Die Vorteile des erfindungsgemäßen Verfahrens liegen darin, dass die Palette an einsetzbaren Lösungsmitteln deutlich erweitert werden konnte. Damit besteht die Möglichkeit, bisher auf Grund schlechter Löslichkeiten oder hoher Schmelzpunkte nicht einsetzbare Phenole, zu cyclischen Ketonen in Lösung zu hydrieren. Ein weiterer großer Vorteil ist, dass die in dem beschriebenen Verfahren eingesetzten Katalysatoren mehrfach wieder eingesetzt werden können, ohne dass es zu einem Verlust an Selektivität kommt. Dies reduziert den Edelmetallverbrauch stark und stellt somit einen enormen Kostenvorteil dar.

Als noch weiterer großer Vorteil ist die Durchführbarkeit des erfindungsgemäßen Verfahrens in der Gegenwart von Wasser ansehen. Durch das Entfallen von aufwendigen Trocknungsmaßnahmen wird die Produktivität des Verfahrens erheblich gesteigert.

Die nachfolgenden Beispiele illustrieren die Erfindung ohne diese einzuschränken.

### Beispiele

Die Versuche wurden mit handelsüblichen alkalischen Pd/C-Katalysatoren (3% Pd/C, ca. 50% wasserfeucht) durchgeführt. Die eingesetzten Rohstoffe und Lösungsmittel stammten aus dem Chemikalienhandel.

### Beispiel 1: Herstellung von 4-tert. Butylcyclohexanon

250 g 4-tert-Butylphenol wurden gelöst in 135 g tert.Butanol und dazu wurden 1,8 g eines mit Natriumcarbonat, Lithiumcarbonat oder Natrium- oder Lithiumacetat versetzten Pd/C-Katalysators und 2,4 g Wasser zugesetzt. Man hydrierte bei 160°C/6 bar Wasserstoffdruck. Die Reaktion war innerhalb 2 Stunden abgeschlossen. Die Reaktionslösungen enthielten 89% 4-tert-Butylcyclohexanon.

### Beispiel 2: Herstellung von 4-tert. Butylcyclohexanon

250 g 4-tert-Butylphenol wurden gelöst in 135 g tert.Butanol und dazu wurden 1,8 g eines mit Kaliumcarbonat oder Cäsiumcarbonat versetzten Pd/C-Katalysators zugesetzt. Man hydrierte bei 160°C/6 bar Wasserstoffdruck. Die Reaktion war innerhalb 2 Stunden abgeschlossen. Die Reaktionslösungen enthielten 87% 4-tert-Butylcyclohexanon.

### Beispiel 3: Herstellung von 4-tert. Butylcyclohexanon

250 g 4-tert-Butylphenol wurden gelöst in 135 g tert.Amylalkohol und dazu wurden 1,8 g eines mit Natriumcarbonat, Lithiumcarbonat oder Lithium- oder Natriumacetat versetzten Pd/C-Katalysators zugesetzt. Man hydrierte bei 160°C/6 bar Wasserstoffdruck. Die Reaktion war innerhalb 2 Stunden abgeschlossen. Die Reaktionslösungen enthielten 87% 4-tert-Butylcyclohexanon.

### Beispiel 4: Herstellung von 4-tert. Butylcyclohexanon

250 g 4-tert-Butylphenol wurden gelöst in 135 g 2-Butanol und dazu wurden 1,8 g eines mit Natriumcarbonat, Lithiumcarbonat oder Lithium- oder Natriumacetat versetzten Pd/C-Katalysators und 2,4 g Wasser zugesetzt. Man hydrierte bei 160°C/6 bar Wasserstoffdruck. Die Reaktion war innerhalb 2 Stunden abgeschlossen. Die Reaktionslösungen enthielten 91% 4-tert-Butylcyclohexanon.

### Beispiel 5: Herstellung von 4-tert. Butylcyclohexanon

300 g 4-tert-Butylphenol wurden gelöst in 125 g 4-Methylpentan-2-ol und dazu wurden 2,9 g eines mit Natriumcarbonat, Lithiumcarbonat oder Natrium- oder Lithiumacetat versetzten Pd/C-Katalysators zugesetzt. Man hydrierte bei 160°C/6 bar Wasserstoffdruck. Die Reaktion war innerhalb 1 Stunde abgeschlossen. Die Reaktionslösungen enthielten 94 bis 96% 4-tert-Butylcyclohexanon.

### Beispiel 6: Herstellung von 4-Methoxycyclohexanon

250 g 4-Methoxyphenol wurden in 125 g tert-Butanol bei 150°C/5 bar Wasserstoffdruck an 2,9 g eines Pd/C-Katalysators, der mit Natrium- oder Lithiumcarbonat oder Natrium- oder Lithiumacetat versetzt worden war, innerhalb 1,7 h hydriert. Die Hydrierlösung enthielt 93% 4-Methoxycyclohexanon.

### Beispiel 7: Herstellung von 4-Methoxycyclohexanon

250 g 4-Methoxyphenol wurden in 125 g 4-Methylpentan-2-ol bei 140°C/4 bar Wasserstoffdruck innerhalb 3 h an einem mit Lithium- oder Natriumcarbonat oder Lithium- oder Natriumacetat versetzten Pd/C-Katalysators hydriert. In der Hydrierlösung lagen 92 bis 94% 4-Methoxycyclohexanon vor.

### Beispiel 8: Herstellung von 4-Methylcyclohexanon

250 g p-Kresol wurden in 130 g 2-Butanol gelöst und bei 170°C/6 bar Wasserstoffdruck an 3,2 g eines mit Lithium- oder Natriumcarbonat oder Lithium- oder Natriumacetat versetzten Pd/C-Katalysators hydriert. Die Hydrierzeit lag bei 3 h. Die Reaktionslösung enthielt 93% 4-Methylcyclohexanon.

### Beispiel 9: Herstellung von 3-Methylcyclohexanon

250 g m-Kresol wurden in 144 g 4-Methylpentan-2-ol gelöst und bei 170°C/6 bar Wasserstoffdruck an 3,3 g eines mit Natrium- oder Lithiumcarbonat oder Natrium- oder Lithiumacetat versetzten Pd/C-Katalysators innerhalb von 1,5 h hydriert. Das Reaktionsgemisch enthielt 96% 3-Methylcyclohexanon.

### Beispiel 10: Herstellung von 2-Methylcyclohexanon

250 g o-Kresol wurden in 144 g 4-Methylpentan-2-ol gelöst und bei 170°C/6 bar Wasserstoffdruck an 3,3 g eines mit Natrium- oder Lithiumcarbonat oder Natrium- oder Lithiumacetat versetzten Pd/C-Katalysators hydriert. Die Hydrierzeit lag bei 3,5 h. In der Reaktionslösung detektierte man 95% 2-Methylcyclohexanon.

### Beispiel 11: Herstellung von 4-Methylcyclohexanon

Man löste 250 g p-Kresol in 142 g Pentan-2-ol und hydrierte bei 160°C/6 bar Wasserstoffdruck an 3,3 g eines mit Natrium- oder Lithiumcarbonat oder Natrium- oder Lithiumacetat versetzten Pd/C-Katalysators. Die Reaktionszeit betrug 2,5 h, in der Reaktionslösung lagen 94% 4-Methylcyclohexanon vor.

### Beispiel 12: Herstellung von 4-Methylcyclohexanon

250 g p-Kresol wurden in 144 g Cyclohexanol gelöst und bei 160°C/6 bar Wasserstoffdruck an 3,3 g eines mit Natrium- oder Lithiumcarbonat oder Natrium- oder Lithiumacetat versetzten Pd/C-Katalysators hydriert. Nach einer Hydrierzeit von 3 h enthielt das Reaktionsgemisch 91% 4-Methylcyclohexanon.

### Beispiel 13: Herstellung von 4-Methoxycyclohexanon

250 g 4-Methoxyphenol wurden gelöst in 125 g 4-Methylpentan-2-ol und bei 140°C/3,5 bar Wasserstoffdruck an 3,7 g eines recyclisierten Pd/C-Katalysators, der nur vor seinem Ersteinsatz mit Natrium- oder Lithiumcarbonat oder Natrium- oder Lithiumacetat versetzt worden war, hydriert. Nach einer Hydrierzeit von 4,7 h lagen in der Reaktionslösung 92% 4-Methoxycyclohexanon vor.

### Beispiel 14: Herstellung von 4-tert.Butylcyclohexanon

300 g 4-tert-Butylphenol wurden in 125 g 4-Methylpentan-2-ol gelöst und bei 160°C/6 bar Wasserstoffdruck an 4,0 g eines recyclisierten Pd/C-Katalysators, der nur vor seinem Ersteinsatz mit Natrium- oder Lithiumcarbonat oder Natrium- oder Lithiumacetat versetzt worden war, hydriert. Die Hydrierzeit lag bei 3,5 h. Das Reaktionsgemisch enthielt 94% 4-tert-Butylcyclohexanon.

### Beispiel 15: Herstellung von 4-tert.Butylcyclohexanon

Man löste 300 g 4-tert-Butylphenol in 125 g 4-Methylpentan-2-ol und hydrierte bei 160°C/6 bar Wasserstoffdruck an 2,9 g eines mit Natriumtetraborat vorbehandelten Pd/C-Katalysators. Die Hydrierzeit betrug 50 min, im Reaktionsgemisch lagen 89% 4-tert-Butylcyclohexanon vor.

### Beispiel 16: Herstellung von 4-tert.Butylcyclohexanon

300 g tert-Butylphenol wurden in 125 g 4-Methylpentan-2-ol gelöst und bei 160°C/6 bar Wasserstoffdruck an 1,7 g eines mit basischem Magnesiumcarbonat und Natriumcarbonat versetzten Pd/C-Katalysators hydriert. Die Hydrierzeit lag bei 1,3 h. Das Reaktionsgemisch enthielt 93% 4-tert-Butylcyclohexanon.

### Vergleichsbeispiele:

### Beispiel 17: Hydrierung an einem nicht-alkalisierten Katalysator

250 g 4-Methoxyphenol wurden gelöst in 125 g 4-Methylpentan-2-ol und bei 140°C/3,5 bar Wasserstoffdruck an 2,9 g eines nicht vorher mit einem basischen oder neutralen Salz versetzten Pd/C-Katalysators hydriert. Nach einer Hydrierzeit von 5 h lagen in der Reaktionslösung 77% 4-Methoxycyclohexanon vor.

### Beispiel 18: Hydrierung in Isooctan

20 kg tert-Butylphenol, gelöst in 20 kg Isooctan wurden an 0,071 g eines mit Natriumcarbonat alkalisierten getrockneten Pd/C-Katalysators bei 150°C/5 bar Wasserstoffdruck innerhalb 3 h hydriert. Im Reaktionsgemisch wurden 80% 4-tert-Butylcyclohexanon detektiert.

Setzte man den Katalysator 50% wasserfeucht, d.h. in der handelsüblichen Form ein, so verklumpte er. Es fand keine Wasserstoffaufnahme statt.

## Patentansprüche

1. Verfahren zur Hydrierung von Phenolen mit einer Hydroxylgruppe zu cyclischen Ketonen durch Umsetzung der Phenole mit Wasserstoff in Gegenwart eines Katalysators in einer Lösung enthaltend einen geradkettigen oder verzweigten sekundären oder tertiären aliphatischen Alkohol mit mindestens drei Kohlenstoffatomen und/oder einen cycloaliphatischen Alkohol mit mindestens drei Kohlenstoffatomen, wobei ein auf Kohlenstoff geträgerter Palladium enthaltender Katalysator verwendet wird, der vor dem Einsatz mit einem neutralen oder basischen Salz behandelt worden ist und/oder wobei die Hydrierung in Gegenwart eines geträgerten Palladium enthaltenden Katalysators und eines neutralen oder basischen Salzes erfolgt, mit der Massgabe, dass der Palladium enthaltende Katalysator keine anorganischen und/oder organischen Schwefelverbindungen enthält.

2. Verfahren nach Anspruch 1, **dadurch gekennzeichnet, dass** die Umsetzung bei Temperaturen von 80 bis 250°C, vorzugsweise von 120 bis 250°C, besonders bevorzugt bei 90 bis 200 °C und ganz besonders bevorzugt bei 1.00 bis 180°C erfolgt.

3. Verfahren nach Anspruch 1, **dadurch gekennzeichnet, dass** die Umsetzung bei einem Wasserstoffdruck von 0,5 bis 20 bar, bevorzugt von 0,5 bis 15 bar, und besonders bevorzugt bei 1 bis 10 bar erfolgt.

4. Verfahren nach Anspruch 1, **dadurch gekennzeichnet, dass** ein sekundärer oder tertiärer Alkohol mit drei bis acht Kohlenstoffatomen eingesetzt wird, ganz besonders bevorzugt ein Alkohol aus der Gruppe Isopropanol, 2-Butanol, tert-Butanol, 2-Pentanol, 3-Pentanol, 2-Methylbutan-2-ol, 3-Methylbutan-2-ol, Cyclopentanol, Cyclohexanol, 2-Methylpentan-2-ol, 2-Methylpentan-3-ol, 3-Methylpentan-2-ol, 3-Methylpentan-3-ol, 4-Methylpentan-2-ol, 2,3-Dimethyl-2-butanol, 3,3-Dimethyl2-butanol, 2-Hexanol, 3-Hexanol, 2,2-Dimethyl-3-pentanol, 2,3-Dimethyl-3-pentanol, 2,4-Dimethyl-3-pentanol, 4,4-Dimethyl-2-pentanol, 3-Ethyl-3-pentanol, 2-Methyl-2-hexanol, 2-Methyl-3-hexanol, 5-Methyl-2-hexanol, 2-Heptanol, 3-Heptanol, 2-Octanol, 3-Octanol, 4-Methylheptan-3-ol, 6-Methylheptan-2-ol, 2-Ethylhexanol oder 3,7-Dimethyl-3-octanol.

5. Verfahren nach Anspruch 1, **dadurch gekennzeichnet, dass** die Umsetzung in einer wässrigen alkoholischen Lösung stattfindet, deren Wassergehalt bis zu 20 Gew. %, bezogen auf die Menge an Gesamtlösungmittel, beträgt.

6. Verfahren nach Anspruch 1, **dadurch gekennzeichnet, dass** die Konzentration des Phenols im aliphatischen und/oder cycloaliphatischen Alkohol 10 bis 95 Gew. % beträgt.

7. Verfahren nach Anspruch 1, **dadurch gekennzeichnet, dass** der geträgerte Palladium enthaltende Katalysator 1 bis 15 Gew. %, bevorzugt 1 bis 10 Gew. % und ganz besonders bevorzugt von 1 bis 5 Gew. % an katalytisch aktivem Metall enthält.

8. Verfahren nach Anspruch 1, **dadurch gekennzeichnet, dass** der geträgerte Palladium enthaltende Katalysator vor dem Einsatz mit einem neutralen oder basischen Salz, vorzugsweise mit einer neutralen oder basischen wässrigen Lösung eines Alkali-, Erdalkali- oder Ammoniumsalzes behandelt wird, ganz besonders bevorzugt mit einer neutralen oder basischen wässrigen Lösung, welche die 1- bis 100-fache Menge an Wasser, bezogen auf das Salz, enthält.

9. Verfahren nach Anspruch 8, **dadurch gekennzeichnet, dass** das neutrale oder basische Salz ein Hydroxid, Carbonat, Hydrogencarbonat, Phosphat, Monohydrogenphosphat, Formiat, Acetat, Propionat oder Borat von Lithium, Natrium, Kalium, Rubidium, Cäsium, Beryllium, Magnesium, Calcium, Strontium, Barium oder Ammonium ist, ganz besonders bevorzugt Lithium-, Natrium-, Kalium- oder Ammoniumhydroxid, Natrium-, Kalium- oder Ammoniumhydrogencarbonat, Lithium-, Natrium-, Kalium-, Ammonium- Magnesium oder Calciumcarbonat, Lithium-, Natrium-, Kalium, Ammonium- oder Magnesiumphosphat, Lithium-, Natrium-, Kalium-, Ammonium- oder Calciumformiat, Lithium-, Natrium-, Kalium-, Ammonium- oder Calciumacetat, Lithium-, Natrium-, Kalium-, Ammonium- oder Calciumpropionat oder Lithium-, Natrium-, Kalium- oder Ammoniumborat ist.

10. Verfahren nach Anspruch 1, **dadurch gekennzeichnet, dass** der geträgerte Palladium-Katalysator, bezogen auf das eingesetzte Phenol, in Mengen von 1 x 10⁻⁴ bis 1 Gew.-% (gerechnet jeweils als Pd-Metall) eingesetzt wird.

11. Verfahren nach..Anspruch 1, **dadurch gekennzeichnet, dass** der eingesetzte geträgerte Palladium-Katalysator frisch ist oder bereits ein- oder mehrmals verwendet worden ist.

12. Verfahren nach Anspruch 1, **dadurch gekennzeichnet, dass** man das dem Katalysator zugesetzte neutrale oder basische Salz in Mengen von 0,001 - 5 Mol-%, bezogen auf eingesetztes Phenol, zugibt.

13. Verfahren nach Anspruch 1, **dadurch gekennzeichnet, dass** als Phenol eine Verbindung der Formel (I) eingesetzt wird 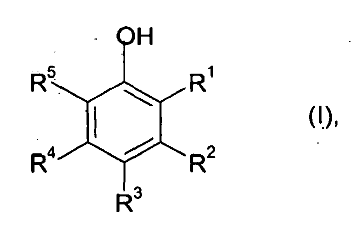 worin R¹, R², R³, R⁴, R⁵ unabhängig voneinander Wasserstoff, C₁-C₁₀-Alkyl, C₃-C₆-Cycloalkyl, C₆-C₁₀-Aryl, C₃-C₁₀-Heteroaryl mit O, N und/oder S als Ring-Heteroätome, C₆-C₁-Aryl-CH₂, C₆-C₁₀-Aryl-O, C₁-C₁₀-Alkoxy, C₃-C₈-Cycloalkoxy, N-C₁-C₄-Alkylamino, N,N-Di-C₁-C₄-alkylamino, C₁-C₄-Acylamino, COOR⁶ mit R⁶ = Wasserstoff, C₁-C₄-Alkyl oder -CH₂-R⁷ mit R⁷ = Hydroxyl, C₁-C₄-Alkyoxy, N-C₁-C₄-Alkylamino oder N, N-Di-C₁-C₄-alkylamino bedeuten, worin R¹, R², R³, R⁴ und R⁵ vorzugsweise unabhängig voneinander für Wasserstoff, C₁-C₁₀-Alkyl, C₁-C₁₀-Alkoxy oder C₁-C₄-Alkylamino stehen und ein bis vier dieser Reste von Wasserstoff verschieden sind, und worin ganz besonders bevorzugt R¹, R², R⁴ und R⁵ unabhängig voneinander für Wasserstoff, C₁-C₁₀-Alkyl, C₁-C₁₀-Alkoxy oder C₁-C₄-Alkylamino stehen.

## Claims

1. A method for hydrogenating phenols with one hydroxyl group to cyclic ketones by reacting the phenols with hydrogen in the presence of a catalyst in a solution comprising a straight-chain or branched secondary or tertiary aliphatic alcohol with at least three carbon atoms and/or a cycloaliphatic alcohol with at least three carbon atoms, wherein a catalyst comprising palladium and supported on carbon is used, which has been treated prior to use with a neutral or basic salt, and/or wherein the hydrogenation takes place in the presence of a supported catalyst comprising palladium and a neutral or basic salt, with the proviso that the catalyst comprising palladium does not comprise any inorganic and/or organic sulfur compounds.

2. The method as claimed in claim 1 wherein the reaction takes place at temperatures of 80 to 250°C, preferably 120 to 250°C, particularly preferably at 90 to 200 °C and very particularly preferably at 100 to 180°C.

3. The method as claimed in claim 1 wherein the reaction takes place at a hydrogen pressure of 0.5 to 20 bar, preferably 0.5 to 15 bar, and particularly preferably at 1 to 10 bar.

4. The method as claimed in claim 1, wherein a secondary or tertiary alcohol with three to eight carbon atoms is used, very particularly preferably an alcohol from the group consisting of isopropanol, 2-butanol, tert-butanol, 2-pentanol, 3-pentanol, 2-methylbutan-2-ol, 3-methylbutan-2-ol, cyclopentanol, cyclohexanol, 2-methylpentan-2-ol, 2-methylpentan-3-ol, 3-methylpentan-2-ol, 3-methylpentan-3-ol, 4-methylpentan-2-ol, 2,3-dimethyl-2-butanol, 3,3-dimethyl-2-butanol, 2-hexanol, 3-hexanol, 2,2-dimethyl-3-pentanol, 2,3-dimethyl-3-pentanol, 2,4-dimethyl-3-pentanol, 4,4-dimethyl-2-pentanol, 3-ethyl-3-pentanol, 2-methyl-2-hexanol, 2-methyl-3-hexanol, 5-methyl-2-hexanol, 2-heptanol, 3-heptanol, 2-octanol, 3-octanol, 4-methylheptan-3-ol, 6-methylheptan-2-ol, 2-ethylhexanol and 3,7-dimethyl-3-octanol.

5. The method as claimed in claim 1, wherein the reaction takes place in an aqueous alcoholic solution, the water content of which is up to 20% by weight, based on the total amount of solvent.

6. The method as claimed in claim 1, wherein the concentration of the phenol in the aliphatic and/or cycloaliphatic alcohol is 10 to 95% by weight.

7. The method as claimed in claim 1, wherein the supported catalyst comprising palladium comprises 1 to 15% by weight, preferably 1 to 10% by weight and very particularly preferably 1 to 5% by weight, of catalytically active metal.

8. The method as claimed in claim 1, wherein the supported catalyst comprising palladium is treated prior to use with a neutral or basic salt, preferably with a neutral or basic aqueous solution of an alkali metal salt, alkaline earth metal salt or ammonium salt, very particularly preferably with a neutral or basic aqueous solution, which comprises 1-fold to 100-fold the amount of water, based on the salt.

9. The method as claimed in claim 8, wherein the neutral or basic salt is an hydroxide, carbonate, hydrogen carbonate, phosphate, monohydrogen phosphate, formate, acetate, propionate or borate of lithium, sodium, potassium, rubidium, cesium, beryllium, magnesium, calcium, strontium, barium or ammonium, very particularly preferably lithium hydroxide, sodium hydroxide, potassium hydroxide or ammonium hydroxide, sodium hydrogen carbonate, potassium hydrogen carbonate or ammonium hydrogen carbonate, lithium carbonate, sodium carbonate, potassium carbonate, ammonium carbonate, magnesium carbonate or calcium carbonate, lithium phosphate, sodium phosphate, potassium phosphate, ammonium phosphate or magnesium phosphate, lithium formate, sodium formate, potassium formate, ammonium formate or calcium formate, lithium acetate, sodium acetate, potassium acetate, ammonium acetate or calcium acetate, lithium propionate, sodium propionate, potassium propionate, ammonium propionate or calcium propionate or lithium borate, sodium borate, potassium borate or ammonium borate.

10. The method as claimed in claim 1, wherein the supported palladium catalyst is used in amounts of 1 x 10⁻⁴ to 1% by weight (calculated in each case as Pd metal), based on the phenol used.

11. The method as claimed in claim 1, wherein the supported palladium catalyst used is fresh or has already been used once or more than once.

12. The method as claimed in claim 1, wherein the neutral or basic salt added to the catalyst is added in amounts of 0.001 - 5 mol%, based on the phenol used.

13. The method as claimed in claim 1, wherein a compound of formula (I) is used as the phenol 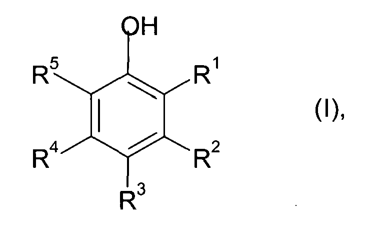 where R¹, R², R³, R⁴, R⁵ independently of one another are hydrogen, C₁-C₁₀-alkyl, C₃-C₆-cycloalkyl, C₆-C₁₀-aryl, C₃-C₁-heteroaryl with O, N and/or S as ring heteroatoms, C₆-C₁₀-aryl-CH₂, C₆-C₁₀-aryl-O, C₁-C₁₀-alkoxy, C₃-C₈-cycloalkoxy, N-C₁-C₄-alkylamino, N,N-di-C₁-C₄-alkylamino, C₁-C₄-acylamino, COOR⁶ where R⁶ = hydrogen, C₁-C₄-alkyl or -CH₂-R⁷ where R⁷ = hydroxyl, C₁-C₄-alkyloxy, N-C₁-C₄-alkylamino or N,N-di-C₁-C₄-alkylamino, where R¹, R², R³, R⁴ and R⁵, independently of one another, are preferably hydrogen, C₁-C₁₀-alkyl, C₁-C₁₀-alkoxy or C₁-C₄-alkylamino and one to four of these residues are different from hydrogen, and wherein very particularly preferably R¹, R², R⁴ and R⁵ independently of one another are hydrogen, C₁-C₁₀-alkyl, C₁-C₁₀-alkoxy or C₁-C₄-alkylamino.

## Revendications

1. Procédé d'hydrogénation de phénols avec un groupe hydroxyle en cétones cycliques par conversion des phénols avec de l'hydrogène en présence d'un catalyseur dans une solution contenant un alcool aliphatique secondaire ou tertiaire, à chaîne droite ou ramifié avec au moins trois atomes de carbone et/ou un alcool cycloaliphatique avec au moins trois atomes de carbone et/ou un alcool cycloaliphatique avec au moins trois atomes de carbone, dans lequel un catalyseur contenant du palladium supporté sur du carbone est utilisé, lequel a été traité avant l'utilisation avec un sel neutre ou basique et/ou dans lequel l'hydrogénation s'effectue en présence d'un catalyseur contenant du palladium supporté et d'un sel neutre ou basique à condition que le catalyseur contenant du palladium ne contienne aucun composé soufré inorganique et/ou organique.

2. Procédé selon la revendication 1, **caractérisé en ce que** la conversion s'effectue à des températures de 80 à 250°C, de préférence de 120 à 250°C, de manière particulièrement préférée à 90 jusqu'à 200°C et de manière tout particulièrement préférée à 100 jusqu'à 180°C.

3. Procédé selon la revendication 1, **caractérisé en ce que** la conversion s'effectue à une pression d'hydrogène de 0,5 à 20 bars, de préférence de 0,5 à 15 bars et de manière particulièrement préférée à 1 jusqu'à 10 bars.

4. Procédé selon la revendication 1, **caractérisé en ce qu'**un alcool secondaire ou tertiaire avec trois à huit atomes de carbone est utilisé, de manière tout particulièrement préférée un alcool parmi le groupe isopropanol, 2-butanol, tert-butanol, 2-pentanol, 3-pentanol, 2-méthylbutan-2-ol, 3-méthylbutan-2-ol, cyclopentanol, cyclohexanol, 2-méthylpentan-2-ol, 2-méthylpentan-3-ol, 3-méthylpentan-2-ol, 3-méthylpentan-3-ol, 4-méthylpentan-2-ol, 2,3-diméthyl-2-butanol, 3,3-diméthyl-2-butanol, 2-hexanol, 3-hexanol, 2,2-diméthyl-3-pentanol, 2,3-diméthyl-3-pentanol, 2,4-diméthyl-3-pentanol, 4,4-diméthyl-2-pentanol, 3-éthyl-3-pentanol, 2-méthyl-2-hexanol, 2-méthyl-3-hexanol, 5-méthyl-2-hexanol, 2-heptanol, 3-heptanol, 2-octanol, 3-octanol, 4-méthylheptan-3-ol, 6-méthylheptan-2-ol, 2-éthylhexanol et 3,7-diméthyl-3-octanol.

5. Procédé selon la revendication 1, **caractérisé en ce que** la conversion a lieu dans une solution alcoolique aqueuse dont la teneur en eau va jusqu'à 20 % en poids par rapport à la quantité de solvant total.

6. Procédé selon la revendication 1, **caractérisé en ce que** la concentration du phénol dans l'alcool aliphatique et/ou cycloaliphatique va de 10 à 95 % en poids.

7. Procédé selon la revendication 1, **caractérisé en ce que** le catalyseur contenant du palladium supporté contient 1 à 15 % en poids, de préférence 1 à 10 % en poids et de manière tout particulièrement préférée de 1 à 5 % en poids de métal actif du point de vue catalytique.

8. Procédé selon la revendication 1, **caractérisé en ce que** le catalyseur contenant du palladium supporté est traité avant l'utilisation avec un sel neutre ou basique, de préférence avec une solution aqueuse neutre ou basique d'un sel alcalin, alcalino-terreux ou d'ammonium, de manière tout particulièrement préférée avec une solution aqueuse neutre ou basique qui contient la quantité simple à centuple en eau par rapport au sel.

9. Procédé selon la revendication 8, **caractérisé en ce que** le sel neutre ou basique est un hydroxyde, carbonate, hydrogénocarbonate, phosphate, monohydrogénophosphate, formiate, acétate, propionate ou borate de lithium, sodium, potassium, rubidium, césium, béryllium, magnésium, calcium, strontium, baryum ou d'ammonium, de manière tout particulièrement préférée l'hydroxyde de lithium, sodium, potassium ou d'ammonium, l'hydrogénocarbonate de sodium, potassium ou d'ammonium, le carbonate de lithium, sodium, potassium, d'ammonium, de magnésium ou calcium, le phosphate de lithium, sodium, potassium, d'ammonium ou de magnésium, le formiate de lithium, sodium, potassium, d'ammonium ou de calcium, l'acétate de lithium, sodium, potassium, d'ammonium ou de calcium, le propionate de lithium, sodium, potassium, d'ammonium ou de calcium ou le borate de lithium, sodium, potassium ou d'ammonium.

10. Procédé selon la revendication 1, **caractérisé en ce que** le catalyseur de palladium supporté est utilisé en des quantités de 1 x 10⁻⁴ à 1 % en poids (calculées à chaque fois comme métal de Pd) par rapport au phénol utilisé.

11. Procédé selon la revendication 1, **caractérisé en ce que** le catalyseur de palladium supporté utilisé est frais ou a déjà été utilisé une ou plusieurs fois.

12. Procédé selon la revendication 1, **caractérisé en ce que** l'on ajoute le sel neutre ou basique ajouté au catalyseur en des quantités de 0,001 à 5 % en mole par rapport au phénol utilisé.

13. Procédé selon la revendication 1, **caractérisé en ce qu'**un composé de la formule (I) est utilisé comme phénol 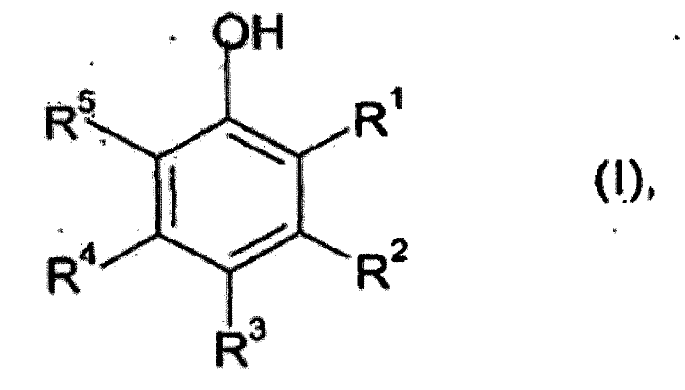 dans laquelle R¹, R², R³, R⁴, R⁵ signifient indépendamment les uns des autres un groupement hydrogène, C₁-C₁₀-alkyle, C₃-C₆-cycloalkyle, C₆-C₁₀-aryle, C₃-C₁₀-hétéroaryle avec O, N et/ou S comme hétéroatomes cycliques, C₆-C₁₀-aryle-CH₂, C₆-C₁₀-aryle-O, C₁-C₁₀-alkoxy, C₃-C₈-cycloalkoxy, N-C₁-C₄-alkylamino, N,N-di-C₁-C₄-alkylamino, C₁-C₄-acylamino, COOR⁶ avec R⁶ = hydrogène, C₁-C₄-alkyle ou -CH₂-R⁷ avec R⁷ = hydroxyle, C₁-C₄-alkyloxy, N-C₁-C₄-alkylamino ou N,N-di-C₁-C₄-alkylamino, dans laquelle R¹, R², R³, R⁴ et R⁵ représentent de préférence indépendamment les uns des autres un groupement hydrogène, C₁-C₁₀-alkyle, C₁-C₁₀-alkoxy ou C₁-C₄-alkylamino et un à quatre de ces radicaux sont différents de l'hydrogène, et dans laquelle de manière tout particulièrement préférée R¹, R², R⁴ et R⁵ représentent indépendamment les uns des autres l'hydrogène, C₁-C₁₀-alkyle, C₁-C₁₀-alkoxy ou C₁-C₄-alkylamino.
